Europäisches Patentamt

European Patent Office

Office européen des brevets

(11) Veröffentlichungsnummer: **0 280 032**
**A2**

(12) **EUROPÄISCHE PATENTANMELDUNG**

(21) Anmeldenummer: **88100716.5**

(22) Anmeldetag: **20.01.88**

(51) Int. Cl.4: **C12N 9/14** , A23C 19/032 ,
//(C12N9/14,C12R1:465)

Claim for the following Contracting State:ES

(30) Priorität: **23.01.87 DE 3701835**

(43) Veröffentlichungstag der Anmeldung:
**31.08.88 Patentblatt 88/35**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lotz, Andreas, Dr.**
**Coventrystrasse 6**
**D-6230 Frankfurt am Main 80(DE)**
Erfinder: **Wöhner, Gerhard Dr.**
**Flörsheimer Strasse 27**
**D-6093 Flörsheim am Main(DE)**
Erfinder: **Klug, Cristian**
**Friedrich-Ebert-Strasse 15**
**D-6231 Schwallbach am Taunus(DE)**
Erfinder: **Lück, Erich, Dr.**
**Robert-Stolz-Strasse 102**
**D-6232 Bad Soden am Taunus(DE)**

(54) **Bakterienlysierendes Enzymprodukt aus Streptomyceten, seine Herstellung und seine Verwendung zur Konservierung von Käse.**

(57) Bakterienlysierende Enzymprodukte aus Streptomyceten können, von Protease befreit, zur Konservierung von Käse verwendet werden. Überraschend zeigte sich, daß das Enzymprodukt aus Streptomyceten in seiner lysierenden Wirkung auf sporenbildende Anaerobier aktiver ist als die entsprechenden aus Sekreten von Tieren und Pflanzen gewonnenen Produkte. Außerdem wirkt das Streptomycetenprodukt auch auf gramnegative Keime und kann so beispielsweise auch zur Verhinderung der Frühblähung eingesetzt werden.

EP 0 280 032 A2

## Bakterienlysierendes Enzymprodukt aus Streptomyceten, seine Herstellung und seine Verwendung zur Konservierung von Käse

In der Französischen Patentschrift 80 03 321 wird der Einsatz von lysozymartigen Produkten aus Sekreten von Tieren und Pflanzen zur Verhinderung der Spätblähung in verschiedenen Käsesorten dargestellt. Die Spätblähung äußert sich im Verlauf der Käsereifung durch eine Verstoffwechselung der anwesenden Milchsäure unter Gasbildung, was zu - schweren Texturfehlern führt. Die Sporen der anaeroben Mikroorganismen, die diese Spätblähung verursachen, werden durch die genannten Enzymprodukte bei der Auskeimung lysiert.

Die Gewinnung eines bakterienlysierenden Enzymprodukts aus Streptomyceten ist bekannt und beispielsweise in den Deutschen Offenlegungsschriften 20 11 935, 20 40 444, 21 46 597 und 34 40 735 beschrieben. Allerdings läßt sich dieses aus Streptomyceten gewonnene Enzymprodukt nicht ohne weiteres zur Konservierung von Käse verwenden.

Überraschend hat sich herausgestellt, daß das Streptomyceten-Enzymprodukt, wenn es beispielsweise nach der Deutschen Offenlegungsschrift 34 40 735 hergestellt wird, mit Proteasen vergesellschaftet ist und nur schwer von diesen zu trennen ist. Es wurde nun gefunden, daß mit Hilfe des bakterienlysierenden, proteasefreien Enzymprodukts aus Streptomyceten Käseprodukte mit besserem Erfolg konserviert werden können als mit entsprechenden Produkten aus Sekreten von Tieren und Pflanzen. Nicht nur die Spätblähung wird effektiver verhindert, sondern es wurde auch gefunden, daß vegetative gramnegative Keime, beispielsweise E. coli, oder auch pathogene Bakterien, z.B. Salmonella, Shigella, Campylobacter oder Listeria ebenfalls durch das Enzymprodukt am Wachstum gehindert werden. Diese Tatsache ist von besonderer Wichtigkeit im Hinblick auf die Konservierung von aus Rohmilch hergestellten Käsen. In den USA traten teilweise tödlich verlaufende Vergiftungen auf durch den Verzehr von Weichkäse, der mit Listeria monocytogenes kontaminiert war. Außerdem kann mit Hilfe des Enzymprodukts aus Streptomyceten, die durch E. coli hervorgerufene Frühblähung unterdrückt werden. Käse läßt sich also auf diese Weise mit niedriger Ausgangskontamination herstellen und ist dadurch insgesamt besser lagerfähig.

Die Erfindung betrifft somit:

1. Ein bakterienlysierendes Enzymprodukt, erhältlich durch Fermentation von Bakterien der Gattung Streptomyces, das durch eine spezifische lytische Aktivität von 20 000 bis 60 000 U/mg Protein gekennzeichnet ist.

2. Das Verfahren zur Herstellung des unter 1 definierten bakterienlysierenden Enzymprodukts, das dadurch gekennzeichnet ist, daß man

a) ein Bakterium aus der Gattung Streptomyces in einem Nährmedium kultiviert und

b) das im Kulturüberstand befindliche genannte Enzymprodukt durch Reinigung mit Hilfe der Ionenaustauscherchromatographie isoliert.

3. Die Verwendung von bakterienlysierendem Enzymprodukt, wie unter 1. definiert, zur Konservierung von Käseprodukten.

Im folgenden wird die Erfindung im einzelnen, insbesondere in ihren bevorzugten Ausführungsformen, erläutert. Weiterhin wird die Erfindung in den Patentansprüchen definiert.

Es kann grundsätzlich jedes lysozymartige Produkt aus Streptomyceten erfindungsgemäß Anwendung finden. In einfach zusammengesetzten Kulturmedien ist mit Hilfe von Streptomyceten bei einer Fermentationszeit von 1 bis 7 Tagen eine hohe Ausbeute an bakterienlysierendem Enzymprodukt erzielbar. Vorzugsweise wird jedoch das gemäß der Deutschen Offenlegungsschrift 34 40 735 aus Streptomyces coelicolor DSM 3030 sowie dessen Varianten und Mutanten gewonnene bakterienlysierende Enzymprodukt eingesetzt.

Besonders bewährt hat sich für die Züchtung von Streptomyces coelicolor DSM 3030 ein Zusatz von Zuckerrübenmelasse in einer Menge von 5 bis 50 g, vorzugsweise 10 bis 20 g, pro Liter Kulturmedium. Eine weitere Ausbeutesteigerung wird erreicht, wenn man dem Kulturmedium Calciumionen in Form leicht löslicher, ungiftiger Calciumsalze zusetzt, vorzugsweise in Form des preiswerten Calciumchlorids. Vorteilhaft ist eine 0,05 bis 1 molare Calciumionenkonzentration, besonders bevorzugt sind Konzentrationen von 100 bis 500 mmol. beispielsweise in Form eines Zusatzes von 0,2 bis 0,5 Gew.-% an Calciumchlorid-Dihydrat.

Da das aus Streptomyceten gewonnene bakterienlysierende Enzymprodukt mit Proteasen vergesellschaftet ist, muß es für den Einsatz als Konservierungsmittel in Käse erst von diesen befreit werden. Diese Auftrennung kann durch Ionenaustauscher-Chromatographie, bevorzugt an - schwach sauren, makroporösen Kationenaustauschern, wie z.B. vernetzte Methacrylate (®Amberlite), erfolgen. Das gewünschte Enzymprodukt bleibt nahezu quantitativ am Austauscherharz gebunden, während der Hauptanteil der Proteasen in der Kulturlösung verbleibt. Durch Elution mit einer 0,05 bis 0,5 molaren Salzlösung, bevorzugt NaCl-Lösung, wird der zurückbleibende Rest der Proteasen von der Säule eluiert. Anschließend wird das bakterienlysierende Enzymprodukt mit einer 0,5 bis 2 molaren Salzlösung, beispielsweise einer NaCl-, Acetat-oder Lactatlösung, von der Säule desorbiert.

Durch anschließende Membranfiltration, bevorzugt Ultrafiltration, beispielsweise auf Celluloseacetatmembranen mit einer Durchlässigkeit von 5000 bis 10000 Dalton, wird das Produkt entsalzt und aufkonzentriert. Das Enzymkonzentrat ist in dieser Form lagerstabil und kann bereits so als Konservierungsmittel eingesetzt werden. Es kann dann noch getrocknet werden, bevor es als Konservierungsmittel in Käse eingesetzt wird. Man erhält erfindungsgemäß ein Enzymprodukt mit einer spezifischen Aktivität von ca. 20000 bis 60000 U/mg Protein, bevorzugt von 30000 bis 40000 U/mg Protein.

Das erfindungsgemäße Enzymprodukt kann vorteilhaft zu der Kesselmilch schon vor der Labzugabe gegeben werden. Es ist zwar auch möglich, das Enzym in den fertigen Käsebruch einzuarbeiten, dann ist eine gleichmäßige Verteilung im Bruch jedoch schwieriger.

Das bakterienlysierende Enzymprodukt kann sowohl als wäßrige, konzentrierte Lösung als auch in unterschiedlichen Formulierungen eingesetzt werden. So kann das Enzym beispielsweise auf einen Träger aufgebracht werden, was den Vorteil hat, daß bei der Dicklegung der Milch das Enzym fast quantitativ im Bruch verbleibt. Das "Coaten" des Enzymprodukts hat noch den zusätzlichen Vorteil, daß das Enzym langsam in dem zu konservierenden Medium freigesetzt wird und dadurch eine länger wirksame Aktivität gewährleistet ist. Als Trägermaterialien können Milchproteine eingesetzt werden, die in einem nativen Zustand vorliegen. Träger und Enzym werden im Verhältnis 20 : 1 bis 200 : 1 in Wasser gelöst und anschließend in handelsüblichen Sprühtrocknern miteinander versprüht. Auch zum "Powder-Coaten", beispielsweise nach dem bekannten "Wurster-Prinzip", können wäßrige Lösungen nativer Milchproteine, wie z.B. Caseinate oder Molkenproteine, oder sonstige gebräuchliche Lebensmittelhilfsstoffe, wie Hydrokolloide, Proteine, Fette (Mono-, Di-und Triglyceride), Wachse etc. eingesetzt werden. Coating-Material und Enzym werden in einem Verhältnis von 10 : 1 bis 1 : 20 eingesetzt. Das Coating-Verfahren nach dem Wurster-Prinzip ist in zahlreichen Patenten beschrieben, so z.B. in den US-Patenten 32 41 520, 35 45 120, 36 98 133 oder 3 950 891.

Die Dosierung des Enzymprodukts kann in weiten Bereichen schwanken. Ein Überschuß ist nicht - schädlich, jedoch sollte sinnvollerweise die Dosierung nach der Verunreinigung der Milch bemessen werden. Daher ist es vorteilhaft, vorher nach an sich bekannten Methoden die mikrobielle Verunreinigung der Milch festzustellen und dann die entsprechende Menge des bakterienlysierenden Enzymprodukts zuzusetzen, die ausreicht, um die - schädlichen Mikroorganismen an einer weiteren Vermehrung zu hindern. Die Konzentrationen können so gewält werden, daß sich 1000 bis 15000 U Enzymprodukt, bevorzugt 1000 bis 10000 U, insbesondere 2000 bis 5000 U, jeweils in 1 g Käseprodukt befinden. Nach Zugabe und Verteilung des bakterienlysierenden Enzymprodukts in der Milch oder auch im Käsebruch verfährt man weiter wie gewohnt bei der Käseherstellung. Starter-und Reifungskulturen, mit denen die Milch im Laufe der Käseherstellung beimpft wird, werden nicht geschädigt.

Es können grundsätzlich alle Käsearten, wie z.B. Hartkäse, Schnittkäse, halbfester Schnittkäse, Weichkäse und Frischkäse, mit dem bakterienlysierenden Enzymprodukt aus Streptomyceten behandelt werden. Bevorzugt wird es jedoch bei der Herstellung von Hartkäse, wie beispielsweise Emmentaler, Bergkäse oder Chester. Schnittkäse, wie z.B. Edamer, Gouda oder Tilsiter, und halbfestem Schnittkäse, wie beispielsweise Wilstermarsch, Butterkäse oder Steinbucher, eingesetzt.

Käseprodukte, die das bakterienlysierende Enzymprodukt aus Streptomyceten enthalten, zeigen je nach Käsesorte nach 6-bis 12-wöchiger Lagerung, auch bei höheren Temperaturen bis zu 20°C, keine Verderbserscheinungen. Der Geschmack des so hergestellten Käses ist nicht beeinträchtigt. Hervorzuheben ist außerdem, daß diese Produkte ohne den Zusatz bzw. mit nur geringen Mengen von Kaliumnitrat hergestellt werden können.

Bakterienlysierende Enzymprodukte aus Steptomyceten lassen sich grundsätzlich kostengünstiger herstellen als die genannten Produkte, die aus den Sekreten von Tieren oder Pflanzen gewonnen werden. Eine kostengünstige Herstellung ist Voraussetzung für eine breite Anwendung des Enzyms in der Käseindustrie.

Das Streptomyceten-Enzymprodukt wirkt im Gegensatz zu den vergleichbaren, bekannten Produkten auch gegen gramnegative Verderbniserreger in der Milch. Gegen grampositive Sporenbilder wirkt es effektiver als die anderen genannten Produkte. Es muß bei gleichem Ergebnis vergleichsweise weniger Streptomyceten-Enzymprodukt bei der Käseherstellung verwendet werden als von den bekannten anderen lysozymartigen Produkten.

In den folgenden Beispielen wird die Erfindung weiter detailliert erläutert. Prozentangaben beziehen sich auf das Gewicht, wenn nicht anders angegeben.

**Beispiel 1**

Isolierung und Reinigung des bakterienlysierenden Enzymprodukts

Nach der Fermentation gemäß Beispiel 3 der Deutschen Offenlegungsschrift 34 40 735 wird die Fermenterbrühe mit einer zum Ca$^+$ äquimolaren Menge an Na-Oxalat versetzt, um das im Nährmedium vorhandende Calcium auszufällen. Anschließend wird das Ca-Oxalat mit dem Pilzmyzel abzentrifugiert. Der Kulturüberstand wird mit Essigsäure auf pH 7,0 eingestellt.

300 ml vernetztes Methacrylat (®Amberlite IRC-50) in der H$^+$-Form werden mit 0,05 molarem Na-Acetatpuffer bei pH 6,0 äquilibriert. Nach Einfüllen des Austauschers in eine Chromatographiesäule werden 10 l Kulturüberstand mit 2 bis 3 BV/Stunde (BV = Bettvolumen) auf die Säule gegeben. Durch Wäsche des Austauschers mit 3 bis 5 BV 0,05 molarem Na-Acetatpuffer (pH 6) und 5 bis 10 BV 0,15 mol NaCl in 0,05 molarem Na-Acetatpuffer (pH 6) wird die Protease entfernt. Anschließend wird das bakterienlysierende Enzymprodukt mit 3 bis 4 BV 1 mol NaCl in 0,05 molarem Na-Acetatpuffer (pH 6) eluiert. Vor der Gefriertrocknung wird die enzymhaltige Lösung mittels Ultrafiltration (10000 Dalton) entsalzt und aufkonzentriert. Das so erhaltene Enzymkonzentrat zeigt keine meßbare Proteaseaktivität und hat eine spezifische lytische Aktivität von durchschnittlich 40000 U/mg Protein.

Aktivitätsbestimmung von bakterienlysierendem Enzymprodukt: Zu 2,8 ml einer Suspension von 0,2 mg Micrococcus luteus ATCC 4698 (Boehringer Mannheim) pro ml 0,1 M Natriumacetatpuffer (pH 5,0) werden 0,2 ml bakterienlysierendes Enzymprodukt-haltige Proben pipettiert und bei 25°C die Abnahme der Trübung durch Messung der Extinktion bei 450 nm bestimmt. Als 1 U wird die Extinktionsabnahme von 0,001 Photometer-Skalaeinheiten pro Minute definiert.

**Beispiel 2**

Verwendung des bakterienlysierenden Enzymprodukts als Konservierungsmittel in Käse

Zu 100 l Kesselmilch werden 100 g Säurewecker (Streptococcus diacetylactis und Streptococcus cremoris, mesophile Mischkulturen der Fa. Wiesby, Nibüll) und 50 ml einer 20 %igen wäßrigen CaCl$_2$-Lösung sowie β-Karotin gegeben. Das Gemisch wird auf eine Temperatur von 32°C gebracht.

5 g des bakterienlysierenden Produkts gemäß Beispiel 1 werden in 2 l Kesselmilch gelöst und unter Rühren den 100 l Milch zugefügt. Nach weiterer Zugabe von 120 g des obengenannten Säureweckers wird das Rührwerk abgeschaltet, um ca. 20 Minuten reifen zu lassen. Die Reifung ist beendet, wenn ein pH-Wert von 6,5 bis 6,3 und eine Soxlett-Henkel Zahl von 7 bis 8 erreicht ist.

Danach wird der Milch 30 ml Lab unter Rühren zugegeben. Die Gerinnungszeit beträgt 30 Minuten. Während dieser Zeit wird der Rührer abgestellt.

Anschließend wird der Bruch mit einem Bruchmesser senkrecht und waagerecht geschnitten. Um eine gleichmäßige Bruchgröße zu erhalten, wird wieder ca. 30 Minuten gerührt. Nach dem Abschöpfen der Molke (ca. 40 l) wird der Bruch 30 Minuten mit 30 l warmem Wasser (60°C) gewaschen. Nach dem Abtrennen der Waschmolke wird der Bruch bei 3 bar über einen Zeitraum von 3 bis 4 Stunden gepreßt und 24 Stunden in ein Salzbad (20 % NaCl) gelegt.

Die anschließende Reifung erfolgt 6 Wochen bei 16 bis 20°C. Auch bei längerer Reifung bis zu 12 Wochen sind keine Verderbserscheinungen zu beobachten.

**Beispiel 3**

Fixierung des bakterienlysierenden Enzymprodukts an einen Träger

1000 g eines handelsüblichen nicht-denaturierten Na-Caseinats wird in 49 l Wasser dispergiert. Der pH-Wert dieser Suspension wird mittels Milchsäure auf pH 5,5 eingestellt. Anschließend werden 1000 ml eines Konzentrats des nach Beispiel 1 hergestellten Enzymprodukts mit einer Aktivität von 1000000 U/ml zugesetzt. Die Lösung wird mit einem handelsüblichen Sprühtrockner, der mit einer Zerstäuberdüse ausgestattet ist, bei einer Lufttemperatur von 80°C getrocknet.

**Beispiel 4**

1000 g eines nach Beispiel 1 hergestellten Enzymprodukts (sprühgetrocknet) werden in einem handelsüblichen Powder-Coater (Wirbelbetttrockner mit Wurster-Einrichtung und Bottom-Spray-Vorrichtung) vorgelegt. Bei einer Umgebungsluft von 20 bis 30°C wird die geiche Menge eines Spezialfettes mit definiertem Schmelzpunkt (> 40°C), z.B. ein Kakaobutter-Substitut aus der Schmelze heraus aufgesprüht. Das Endprodukt ist noch rieselfähig.

## Ansprüche

1. Bakterienlysierendes Enzymprodukt, erhältlich durch Fermentation von Bakterien der Gattung Streptomyces, gekennzeichnet durch eine Spezifische lytische Aktivität von 20000 bis 60000 U/mg Protein.

2. Bakterienlysierendes Enzymprodukt nach Anspruch 1, gekennzeichnet durch eine spezifische lytische Aktivität von 30000 bis 40000 U/mg Protein.

3. Bakterienlysierendes Enzymprodukt nach Anspruch 1 oder 2, dadurch gekennzeichnet, daß es durch Fermentation von Streptomyces coelicolor DSM 3030, seinen Varianten und Mutanten erhältlich ist.

4. Verfahren zur Herstellung des bakterienlysierenden Enyzmprodukts gemäß einem der Ansprüche 1 oder 2, dadurch gekennzeichnet, daß man

a) ein Bakterium aus der Gattung Streptomyces in einem Nährmedium kultiviert und

b) das im Kulturüberstand befindliche genannte Enzymprodukt durch Reinigung mit Hilfe der Ionenaustauscherchromatographie isoliert.

5. Verfahren nach Anspruch 4, dadurch gekennzeichnet, daß Streptomyces coelicolor DSM 3030 kultiviert wird.

6. Verwendung von bakterienlysierendem Enzymprodukt nach einem oder mehreren der Ansprüche 1 bis 3 zur Konservierung von Käseprodukten.

7. Verwendung nach Anspruch 6, dadurch gekennzeichnet, daß man das Enzymprodukt aus Streptomyces coelicolor DSM 3030 sowie aus dessen Varianten und Mutanten einsetzt.

8. Verwendung nach Anspruch 6 oder 7, dadurch gekennzeichnet, daß die Zugabe des Enzymprodukts so erfolgt, daß sich davon 1000 bis 15000 U in 1 g Käseprodukt befinden.

9. Verwendung nach einem oder mehreren der Ansprüche 6 bis 8, dadurch gekennzeichnet, daß man das bakterienlysierende Enzymprodukt auf einen Träger aufzieht.

10. Verwendung nach einem oder mehreren der Ansprüche 6 bis 8, dadurch gekennzeichnet, daß das bakterienlysierende Enzymprodukt mit einem für Lebensmittel geeigneten Hilfsstoff in pulverförmiger Form eingekapselt wird.

11. Verwendung nach einem oder mehreren der Ansprüche 6 bis 10, dadurch gekennzeichnet, daß das Enzymprodukt in Hartkäse, Schnittkäse und halbfestem Schnittkäse eingesetzt wird.

Patentansprüche für den folgenden Vertragsstaat: ES

1. Verfahren zur Herstellung eines bakterienlysierenden Enzymprodukts mit einer spezifischen lytischen Aktivität von 20000 bis 60000 U/mg Protein, dadurch gekennzeichnet, daß man

a) ein Bakterium aus der Gattung Streptomyces in einem Nährmedium kultiviert und

b) das im Kulturüberstand befindliche genannte Enzymprodukt durch Reinigung mit Hilfe der Ionenaustauscherchromatographie isoliert.

2. Verfahren nach Anspruch 1, dadurch gekennzeichnet, daß ein bakterienlysierendes Enzymprodukt mit einer spezifischen lytischen Aktivität von 30000 bis 40000 U/mg Protein hergestellt wird.

3. Verfahren nach Anspruch 1 oder 2, dadurch gekennzeichnet, daß das bakterienlysierende Enzymprodukt durch Fermentation von Streptomyces coelicolor DSM 3030, seinen Varianten und Mutanten hergestellt wird.

4. Bakterienlysierendes Enzymprodukt, mit einer spezifischen lytischen Aktivität von 20000 bis 60000 U/mg Protein, erhältlich gemäß Verfahren nach einem oder mehreren der Ansprüche 1 bis 3.

5. Verwendung von bakterienlysierendem Enzymprodukt erhältlich nach einem oder mehreren der Ansprüche 1 bis 3 zur Konservierung von Käseprodukten.

6. Verwendung nach Anspruch 5, dadurch gekennzeichnet, daß die Zugabe des Enzymprodukts so erfolgt, daß sich davon 1000 bis 15000 U in 1 g Käseprodukt befinden.

7. Verwendung nach Anspruch 5 oder 6, dadurch gekennzeichnet, daß man das bakterienlysierende Enzymprodukt auf einen Träger aufzieht.

8. Verwendung nach einem oder mehreren der Ansprüche 5 bis 7, dadurch gekennzeichnet, daß das bakterienlysierende Enzymprodukt mit einem für Lebensmittel geeigneten Hilfsstoff in pulverförmiger Form eingekapselt wird.

9. Verwendung nach einem oder mehreren der Ansprüche 5 bis 8, dadurch gekennzeichnet, daß das Enzymprodukt in Hartkäse, Schnittkäse und halbfestem Schnittkäse eingesetzt wird.